# EUROPEAN PATENT APPLICATION

(11) **EP 4 116 703 A1**
(43) Date of publication of application: **11.01.2023**
(21) Application number: 21183681.2
(22) Date of filing: 05.07.2021
(51) Int. Cl.: G01N 21/64, G01N 33/542, C12N 7/00, C12N 15/10, G01N 33/68

(54) **PHAGE-BASED METHOD FOR DETECTING BIOMARKERS**

(71) Applicant: Aqsens Health Oy, 20520 Turku (FI)
(72) Inventor: KULPAKKO, Janne, 20520 Turku (FI); LEHTI, Vilhelmiina, 20520 Turku (FI); TEIMONEN, Timo, 20520 Turku (FI)
(74) Representative: Laine IP Oy

(57) **Abstract**

According to an example aspect of the present invention, there is provided a method for determining the presence of a biomarker for disease in a pretreated biological sample comprising the steps of diluting the sample, contacting the sample with a europium label, a terbium label or a samarium label, and a dye, contacting the sample with a phage, incubating the sample, measuring luminescence emission intensity or absorption emission intensity, and determining the presence of the biomarker based on the measurement of the luminescence emission intensity and/or the absorption emission intensity..

## Description

### FIELD

The present invention relates to a phage-based method for detecting biomarkers. Further, the present invention relates to a phage-based method for determining the presence of biomarkers for disease in a biological sample.

### BACKGROUND

Disease state in a patient is typically assessed using antibody assays with known biomarkers, namely proteins. Antibody assays for diseases like prostate cancer are often ambiguous and marker proteins are frequently elevated for other reasons that are unknown, resulting in false positives, but equally as important false negatives when assessing disease state, e.g. in prostate cancer patients.

Prostate cancer is the most common cancer type for men. Overdiagnosis of prostate cancer may result in overtreatment. This often causes long-term side effects that impair the quality of life. The challenge is to separate the type of prostate cancer that grows very slowly and causes minimal harm from the aggressive or metastatic prostate cancer types. Typical prostate cancer diagnosis covers methods of prostate specific antigen (PSA) detection, digital rectal examination, cystoscopy and magnetic resonance imaging (MRI). PSA detection is the easiest and the most cost-effective, as it can be measured from a blood sample in a laboratory. However, a drawback of PSA detection is that the amount of PSA in the blood can rise also from several other reasons e.g. in benign conditions and therefore the detection gives high false positive results. Such false positive results then lead to additional diagnosis and unnecessary treatments, not to mention mental stress. None of the above listed methods are suitable for the population level screening of prostate cancer.

Kulpakko et al. (Anal Biochem. 2019 Apr 1;570:21-26) have described the use of sensitive lanthanide labels with phages opening a new track for developing scalable screening tests for hospital samples. Lanthanide luminescence is "tuned" to differentiate samples using non-covalently interacting, luminescence modulating components such as luminescence quenching dyes.

### SUMMARY OF THE INVENTION

It is an object of the present invention to provide a phage-based method for determining the presence of a biomarker in a biological sample. A further object is to provide a kit for determining the presence of a biomarker in a biological sample.

Thus, according to a first aspect, the present invention relates to determining the presence of a biomarker for disease in a pretreated biological sample comprising the steps of diluting the sample, contacting the sample with a label comprising a europium ion, a terbium ion or a samarium ion, and a dye, contacting the sample with a phage, incubating the sample, measuring luminescence emission intensity or absorption emission intensity, and determining the presence of the biomarker based on the measurement of the luminescence emission intensity or the absorption emission intensity.

According to a second aspect, the present invention relates to a method for visually determining the presence of a biomarker in a biological sample comprising the steps of diluting the sample, contacting the sample with a label comprising a europium ion, a terbium ion or a samarium ion, and a dye, contacting the sample with a phage, incubating the sample, observing the colour of the sample, and determining the presence of a biomarker based on the colour.

According to a third aspect, the present invention relates to a kit for visually determining the presence of a biomarker for disease in a sample, said kit comprising means for taking a sample, means for preparing the sample, means for filtering the sample, a receptacle containing a label, a dye, and a phage for determining the presence of a biomarker for disease, and a colour scale for comparing a test result to a control.

### BRIEF DESCRIPTION OF THE DRAWINGS

FIGURE 1 shows relative absorbance increases as the amount of CRP detected with a CRP specific phage increases in a sample according to at least some embodiments of the invention.
FIGURE 2 show TRF luminescence decreases as the amount of CRP detected with a CRP specific phage increases in a sample according to at least some embodiments of the invention
FIGURE 3 shows the difference in luminescence signal strength or intensity in non-lethal prostate cancer samples and lethal prostate cancer samples detected with a lethal prostate cancer specific phage according to at least some embodiments according to the present invention.
FIGURE 4 shows the difference in PSA in non lethal prostate cancer samples compared to lethal prostate cancer samples, measured by a PSA test.
FIGURE 5 illustrates colour intensity and disorder of dyes decreases as rounds of biopanning increase.
FIGURE 6 shows a biopanning process for the provision of phages according to at least some embodiments of the present invention.
FIGURE 7 shows raw luminescence data from a panel of 88 urine samples. The black bars show signals related to lethal cases and the other ladder-type bars show variance of typical signals.

### EMBODIMENTS

### DEFINITIONS

In the present context, the term "pretreated biological sample" means a biological that is not a virgin sample used untouched, but a sample that has been treated, for example, by filtration, sonification, centrifugation, dilution, freeze drying, and/or desiccation.

Thus pretreatment includes filtration sonification, centrifugation, dilution, freeze drying and desiccation.

TRF luminescence stands for time-resolved fluorescence. TRF luminescence uses fluorophores with a distinctive fluorescence lifetime to avoid interference caused by molecules with a very different fluorescence lifetime or other factors (most importantly excitation light) meaning the fluorescence of a sample monitored as a function of time after excitation by a pulse of light.

For the purposes of the present invention, the term ultracentrifugation means centrifugation at speeds in the range of 40,000 to 120,000 rpm. This speed is capable of gradent separation of at least 1 to 1000 nm size particles from the surrounding media

Room temperature shall be taken to mean a temperature in the range of 20°C to 25°C measured at normal atmospheric pressure.

Lignin-like polymer shall be taken to mean lignin or a polymer like lignin meaning an amorphous, irregular three dimensional and highly branched phenolic polymer.

Phage, for the purposes of the present invention shall be taken to mean a whole bacteriophage that is suitable for biopanning processes to determine the phage's, which include biophages and biosensing phages, affinity for a biomarker in a disease sample.

Dye and quencher shall be taken to be alternative descriptions of the same molecule for example brilliant green is a dye that quenches europium luminescence.

It is an aim of the present invention to overcome at least some of the problems associated with the prior art and provide a simple, quick and reliable method for determining the presence of a biomarker in a biological sample. By means of embodiments of the present invention, it has surprisingly been found that a phage, particularly a biosensing phage, typically selected on the basis of its affinity for a dye, may be trained in a biopanning process to detect a biomarker associated with a disease.

In a method according to at least some embodiments of the invention a two stage biopanning process is utilised in order to create trained phages capable of capturing quencher dye and finally disease specific molecules interacting with phage-dye complexes. As the molecules in the disease sample interfere with the phage, quenching dye is repelled from the phage and a difference in the lanthanide luminescence is observed. The method is wash free, fast and cost-effective and presents an alternative for screening of faecal and urine samples changes due to disease biomarkers. By means of the invention, it has surprisingly been found that any biomolecules can be detected without laborious development and production of antibodies, immobilizing or labelling assay reagents. Further, the dye-phage-biomarker complex is visible with the naked eye.

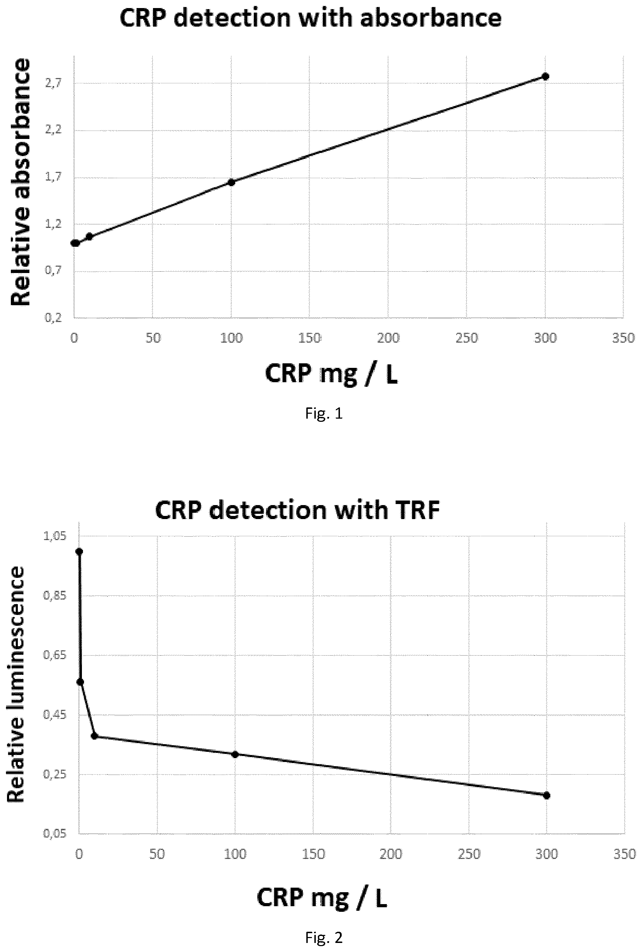

FIGURE 1 illustrates the principle of embodiments of the invention. CRP (C-reactive protein) was selected as a model protein that was used as a target for brilliant green binding phages in biopanning procedures. CRP was selected to simulate a biomarker, *i.e* in this experimental case the biomarker to which the phage is trained is known, whereas in actual disease cases, the phage is trained towards disease samples containing known or unknown biomarkers. As the concentration of CRP in the sample increases, relative absorbance at 623 nm increases. As there is more CRP in the sample, more CRP binds to the phage and dye is released increasing the colour intensity in a sample, which is visible to the naked eye.

FIGURE 2 illustrates the same principle of embodiments of the invention, but measurement of the presence of CRP in the sample is carried out by TRF luminescence. As the amount of CRP in the sample increases, more CRP binds to the phage and dye is released. The free moving dye molecules quench or shut down the label resulting in a low luminescence signal.

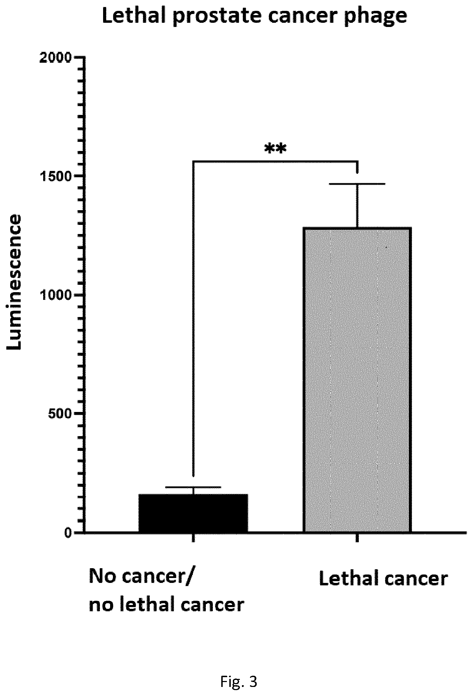

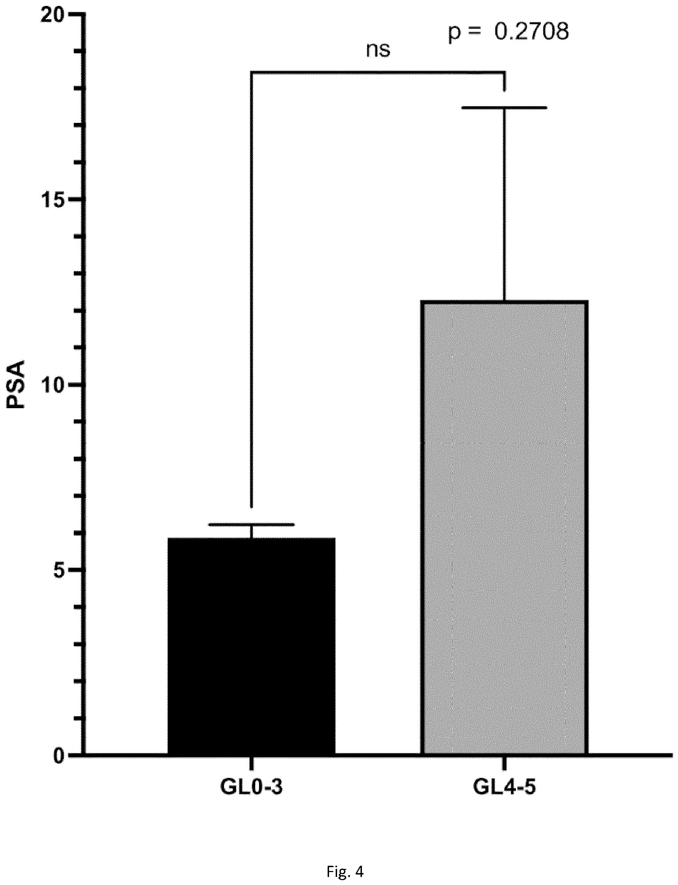

FIGURE 3 illustrates the difference in luminescence in lethal prostate cancer samples compared to non-lethal prostate cancer/ no cancer samples. A phage based method with chemical modulator resazurin provided clearly better *p*-values (p = 0.0014) than a commercial PSA assay (p = 0.2708), which is illustrated in FIGURE 4. Figures 3 and 4 illustrate that the combination of phages and quenching dye molecules in a biosensing platform is well-suited to detecting a variety of different disease states that lack clear biomarker candidates.

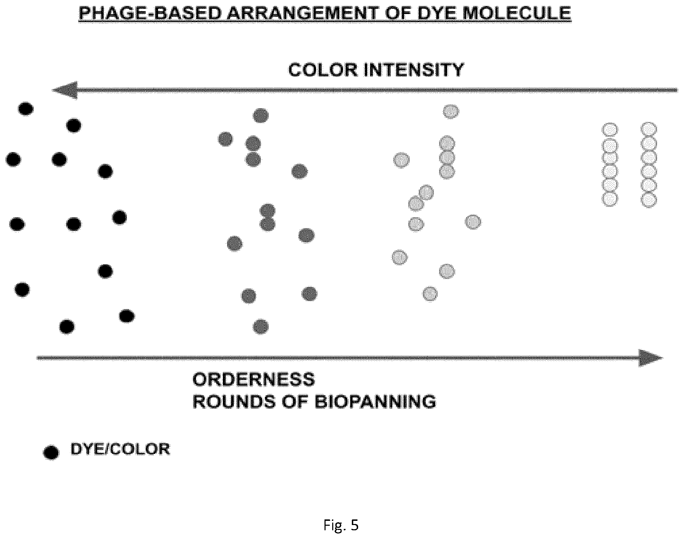

FIGURE 5 illustrates the phage-based arrangement of dye molecules. As the number of rounds of biopanning increase, more dye or colour becomes bound to phages and the colour intensity therefore decreases. Randomly distributed dye molecules have normal colour intensity. Phage affinity towards dye decreases color intensity as dye arranges on the surface of the phage (phages not visible in the figure). Each biopanning round strengthens the affinity towards dye molecules and the solution turns more colorless. This is also seen with the change of absorbance peak of the dye.

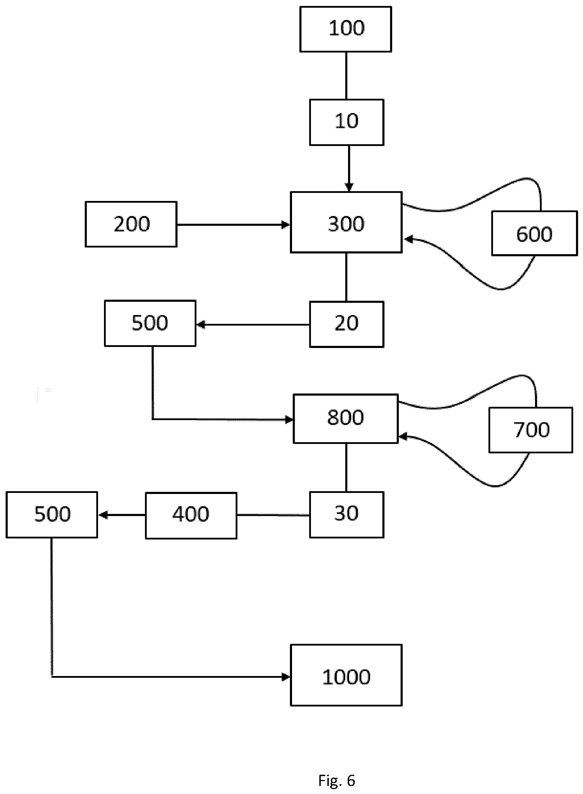

FIGURE 6 illustrates a method of developing a biosensing system. In a first step of a first biopanning process a dye or quencher binding phage (10) is selected from a phage library (100). Dye or quencher molecules (200) which are bound by the selected phage are attached to a substrate (300). The dye or quencher molecules (200) attached to the substrate (300) are then contacted with the phage (10) selected from the phage library (100). After incubating, unbound phage is washed from the substrate (300) in a washing step. Biopanned bound phage (20) is then eluted from the substrate (300) and recovered and amplified to make more copies. These binding incubating and eluting steps may be repeated in a number of stage I biopanning iterations (600). Biopanned dye or quencher binding phages (20) are subjected to luminescence assay testing (500). These biopanned dye or quencher binding phages (20) are directed to a further biopanning process against pooled disease samples (800), in which disease samples in well plates were incubated with the biopanned dye or quencher-binding phages (20) overnight at room temperature before blocking with BSA. This second stage biopanning process may also be repeated in a number of stage II biopanning iterations iterations (700). Biopanned biomarker binding bound phages (30) were harvested and multilplied for luminescence assay testing of the functionality of the assay system (500). Biopanned biomarker binding bound phages (30) that reduce luminescence when tested with a set of samples disease samples (400) are biosensing phages (1000).

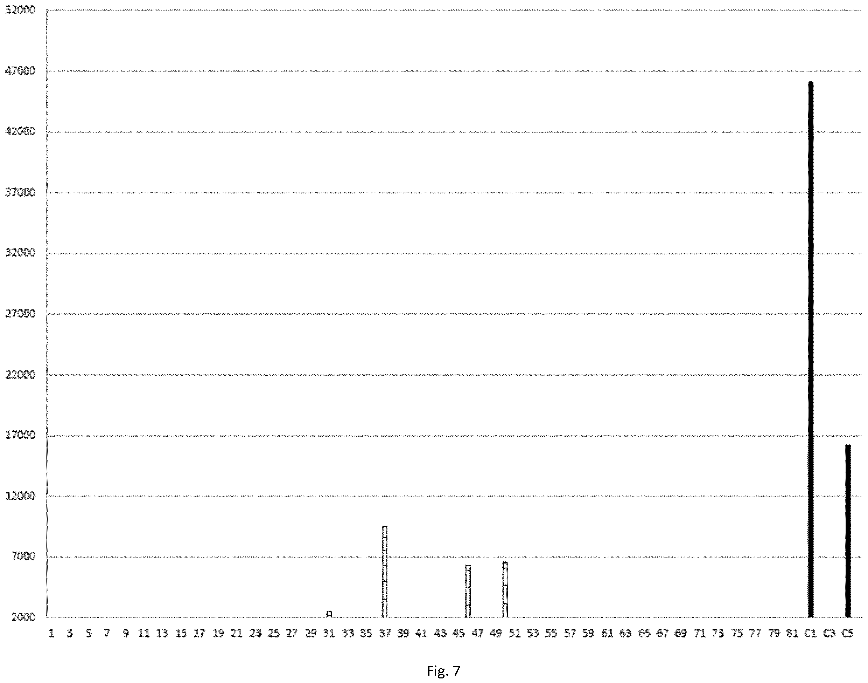

FIGURE 7 illustrates the detection of lethal prostate cancer by means of embodiments of the invention. From a total of 88 samples it can be seen that after contacting the sample with lethal prostate cancer detecting phages according to embodiments of the method, samples containing biomarkers indicating lethal prostate cancer (C1 and C5) luminesce at levels in the range of from about twice to about 23 times the amount of samples that do not contain a biomarker indicating lethal prostate cancer (31, 37, 46, 50).

As mentioned above, embodiments relate to a method for determining the presence of a biomarker for disease in a pretreated biological sample. In an embodiment the method comprises the steps of diluting the sample, contacting the sample with a europium label, a terbium label or a samarium label, each label preferably comprising a europium ion, a terbium ion or a samarium ion, and each label comprising a suitable anion, and a dye, contacting the sample with a phage, incubating the sample, measuring luminescence emission intensity or absorption emission intensity, and determining the presence of the biomarker based on the measurement of the luminescence emission intensity and/or absorption emission intensity. In a preferred embodiment, the label comprises a chloride anion. Thus, in an embodiment the label is selected from the group consisting of europium chloride, terbium chloride or samarium chloride. As is known to a person of skill in the art europium chloride (EuCl₃), terbium chloride (TbCl₃) and samarium chloride (SmCl₂) exist as complexes. For example, europium(III) chloride, 99.99%, CAS Number 10025-76-0, from Sigma-Aldrich and terbium(III) chloride, 99.99%, CAS Number 10042-88-3, from Sigma-Aldrich can be used. The europium chloride label can be used for example as a complex of europium chloride, nitrilotriacetic acid (NTA) (for example 99 %, CAS Number 139-13-9, from Sigma-Aldrich) and trioctylphosphine oxide (TOPO) (for example 99 %, CAS Number 78-50-2, from Sigma-Aldrich).

One possible combination is these three components in a 3:9:9 ratio. Instead of NTA, also 2-thenoyltrifluoroacetone (for example 99 %, CAS Number 326-91-0, from Sigma-Aldrich) or 4,4,4-trifluoro-1-(2-furyl)-1,3-butanedione (for example 99 %, CAS Number 326-90-9, from Sigma-Aldrich can be used.

The concentrations of the various components of a complex of europium chloride 80 nM - 5.0 µM for the europium chloride; 5 nM - 3 µM for TOPO and; 100 nM - 3 µM for NTA. The concentration of europium chloride can thus be for example from 80 nM, 100 nM, 200 nM, 300 nM, 400 nM, 500 nM, 600 nM, 700 nM, 800 nM, 900 nM, 1 µM, 1.5 µM, 2 µM, 2.5 µM, 3 µM, 3.5 µM or 4 µM up to 200 nM, 300 nM, 400 nM, 500 nM, 600 nM, 700 nM, 800 nM, 900 nM, 1 µM, 1.5 µM, 2 µM, 2.5 µM, 3 µM, 3.5 µM, 4 µM, 4.5 µM or 5 µM. The concentration of TOPO can be for example from 5 nM, 10 nM, 15 nM, 20 nM, 25 nM, 30 nM, 35 nM, 40 nM, 45 nM, 50 nM, 55 nM, 60 nM, 65 nM, 70 nM, 75 nM, 80 nM, 100 nM, 200 nM, 300 nM, 400 nM, 500 nM, 600 nM, 700 nM, 800 nM, 900 nM, 1 µM, 1.5 µM or 2 µM up to 15 nM, 20 nM, 25 nM, 30 nM, 35 nM, 40 nM, 45 nM, 50 nM, 55 nM, 60 nM, 65 nM, 70 nM, 75 nM, 80 nM, 100 nM, 200 nM, 300 nM, 400 nM, 500 nM, 600 nM, 700 nM, 800 nM, 900 nM, 1 µM, 1.5 µM, 2 µM, 2.5 µM or 3 µM. The concentration of NTA can be for example from 100 nM, 200 nM, 300 nM, 400 nM, 500 nM, 600 nM, 700 nM, 800 nM, 900 nM, 1 µM, 1.5 µM or 2 µM up to 200 nM, 300 nM, 400 nM, 500 nM, 600 nM, 700 nM, 800 nM, 900 nM, 1 µM, 1.5 µM, 2 µM, 2.5 µM or 3 µM.

In the diluting step, the pretreated sample is preferably diluted in physiological saline, suitably 1 part sample to 50 parts saline. The diluted sample is then contacted with a label and a dye and a biosensor. Suitably, the label, dye and biosensor may contact the sample simultaneously, although the order in which the label, dye and biosensor contact the sample is not important. Preferably, the sample is diluted and then contacted with the label and the phage. The dye may suitably be added during the dilution of the sample. Preferably, the sample is incubated at room temperature, typically in a laboratory shaker, suitably for a period of up to 20 minutes, for example 5 to 15 minutes, preferably for 10 minutes. The sample, may however, remain in incubation for a period of up to 24 hours without detriment to the sample or the result. In some embodiments, the dye reaction enhances for up to 24 hours. Luminescence emission intensity is typically measured in a 400 µs window after a 400 µs delay. Optionally, luminescence emission intensity is measured in a 400 µs +/- 50 µs window after a 400 µs +/- 50 µs delay. Preferably, the luminescence emitted from the label is TRF luminescence. Typically, fluorophores of choice are chelates of lanthanides because their fluorescence time is in the range of microseconds (µs), while other fluorophores make fluorescence for periods in the range of nanoseconds (ns). This µs scale window for measuring fluorescence allows measurements to be performed after both the light of the excitation and the fluorescence of other fluorophores in the sample have completely decayed, thereby greatly reducing the background noise and thus increasing the signal to noise ratio. TRF luminescence may be measured with devices known to those skilled in the art, , for example emitted luminescence may be measured with a Victor 2 multilabel counter or as a further example with a Tecan spark multimode microplate reader. A Victor 2 multilabel counter, or a Tecan spark multimode microplate reader. as well as other devices known to the person of ordinary skill in the art, is also suitable for measuring absorption emission intensity in embodiments, particularly at a wavelength in the range of 620 nm to 640 nm, preferably 623 nm to 635nm.

Further embodiments relate to a further method for determining the presence of a biomarker for disease in a pretreated biological sample, preferably by visual inspection. Thus, an embodiment relates to a method for visually determining the presence of a biomarker in a biological sample comprising the steps of diluting the sample, contacting the sample with a europium label, a terbium label or a samarium label, and a dye, contacting the sample with a phage, incubating the sample, observing the colour of the sample, and determining the presence of a biomarker based on the colour. In an embodiment the label is selected from the group consisting of europium chloride, terbium chloride and samarium chloride. Preferably, the diluting, contacting and incubating steps are carried out in the same way as described in embodiments above.

As will become apparent methods according to embodiments may use a diverse range of biological samples. Thus, in an embodiment the pretreated sample is derived from saliva, sputum, urine, faeces or blood *e.g.* derived from blood serum or blood plasma.

In an embodiment the pretreated sample is obtained by centrifuging urine, saliva or sputum, at a speed in the range of 8000 - 12000 rpm, preferably 10000 rpm for a period of 5 to 20 minutes and recovering a supernatant. The upper limit for the speed of centrifugation is not necessarily 12000 rpm. It is, however, important that ultracentrifugation does not occur. Thus, the upper limit for the speed of centrifucation is, in embodiments, a speed lower than that at which ultracentrifugation takes place.

In a further embodiment the pretreated sample is obtained from faeces. In an embodiment the pretreated sample is obtained by adding 1 part faeces to 5 parts physiological saline to provide a mixture, vortexing the mixture, sonicating the mixture for a period of 5 to 20 minutes at a temperature in the range of 40 - 50°C, centrifuging at speed in the range of 8000 - 12000 rpm, preferably 10000 rpm, for a period of 5 to 20 minutes, recovering a supernatant. The vortexed mixture should not be sonicated for longer than 20 minutes to avoid enzymatic reactions that destroy biomarkers in the sample from taking place. As described above for samples obtained from urine, saliva or sputum, the upper limit for speed of centrifugation is not necessarily 12000 rpm, but for the purposes of embodiments has been selected to avoid ultracentrifugation.

In an embodiment the pretreated sample is obtained from blood, particularly from blood serum or blood plasma. In such an embodiment the pretreated sample is obtained by diluting blood serum or blood plasma in physiological saline in a ratio ranging from 1:10 to 1:1000 by volume, centrifuging at a speed in the range of 8000 to 12000 rpm for a period of 5 to 20 minutes and recovering a supernatant. Similarly to above-described embodiments it is important that ultracentrifugation does not occur, whereby the upper limit of 12000 rpm for centrifugation is selected.

In a particularly preferred embodiment the phage is a biosensing phage provided by biopanning in a process comprising contacting a substrate with a dye to immobilize the dye on the substrate, selecting a dye binding phage from a phage library and contacting the substrate with the selected phage, recovering and amplifying binding phages from the surface of the substrate, contacting the recovered binding phages with the label, the dye and pooled disease samples comprising the biomarker for disease, harvesting and multiplying the phages bound to the biomarker. In one embodiment the substrate is lignin or a lignin-like polymer in the form of chips. The dye that is immobilized on the substrate is the same dye that is contacted with the sample in embodiments of the method of determining the presence of a biomarker for diseases in a pretreated biological sample. The dye binding phage is selected from a phage library based on its affinity for the dye that has been contacted with the substrate, *e.g*. one preferred phage may have an affinity for brilliant green or a brilliant green dye modified at the *ortho, meta,* and/or *para* positions, while a further preferred phage may have an affinity for nile blue A. Unbound phage is washed from the substrate-dye chips with physiological saline and then with buffer. Bound phages are eluted from the chips and recovered to make further copies in an amplification step in which the eluted phage is contacted with host bacteria, *e.g*. with e-coli in a growth medium whereby the amount of phage is amplified, for example, in the region of 10¹³ times. Thus, dye-binding phages are amplified in a selective or controlled evolution process. These dye binding phages are then contacted with the label and pooled disease samples for biopanning. After biopanning, phages bound to disease specific biomarkers present in the disease samples are harvested and multiplied in an amplification step as described above. The biopanning process according to embodiments is illustrated in Figure 6.

In embodiments the phage, preferably the biosensing phage is provided as an aqueous suspension. Optionally, the biosensor may be provided in glycerol.

As described above, a dye is employed in both the method of detecting a biomarker and in the biopanning process. In embodiments the dye is selected from the group consisting of brilliant green, nile blue A, resazurin, and methylene blue. Each of these dyes are able to quench labels comprising europium, terbium and samarium in embodiments.

Further embodiments relate to a kit for visually determining the presence of a biomarker for disease in a sample. In an embodiment the kit comprises means for taking a sample, means for preparing the sample, means for filtering the sample, a receptacle containing a label, a dye, and a phage for determining the presence of a biomarker for disease, and a colour scale for comparing a test result to a control. The means for taking a sample is in an embodiment preferably any non-invasive means, *e.g.* a receptacle for receiving a urine sample or a receptacle for receiving a faeces sample or a receptacle for receiving a sputum or saliva sample or a buccal swab. In an embodiment the means for preparing the sample comprises a syringe. In a further embodiment the means for preparing the sample further comprises a membrane for filtering the sample whereby the sample is filtered to provide a supernatant, which is directed to the receptacle containing the label, dye and phage. In one embodiment the label, dye and phage are all in one receptacle in solid or liquid form. In a further embodiment the label, dye and phage are in more than one receptacle. The contents of said receptacles are then typically mixed with the prepared and filtered sample to determine the presence of a biomarker in the sample.

### EXPERIMENTAL PART

Randomly distributed dye molecules have expected colour characteristics in normal conditions (Figure 5). Phage affinity towards a dye decreases colour intensity as the dye arranges itself on the surface of the phage (phages not visible in the figure). Each biopanning round strengthens the affinity of the phage towards dye molecules and the solution turns more colourless. This is also seen with the change of absorbance peak of the dye.

Dye binders were selected from a phage library. Dye was immobilized on lignin chips and binding phages were selected from the lignin surface (Figure 6). The functionality of the binding phages was studied with Time-resolved fluorescence (TRF) and absorbance measurements (ABS). Binding phages from this first screening were used in a second screening against a pool of patient samples. Phage clones were tested with a sensitive lanthanide label in the presence of dye and patient samples. From the tested group of clones the best interacting phage was enriched. The function of the chosen phage was further tested with individual samples. Finally, the data obtained was compared to the diagnosis of patients provided by other methods.

### Example 1

The lignin chips were rinsed with distilled water and autoclaved 120°C for 60 min before the biopanning experiments.The biopanning system, The Ph.D.-12 phage display peptide library containing 1.5×10 13 plaque-forming units (pfu)/ml had required complexity of 2×10 9 An 10µl aliquot of the random peptide library was incubated with a lignin chips covered with brilliant green at RT for 30 min with gentle shaking in a microcentrifuge tube containing 1 ml of physiological saline. Unbound phage from lignin-brilliant green chips was washed serially with 4 ml of physiological saline and final washing rounds with TBST buffer [0.1% (v/v) Tween-20 in TBS (50mM Tris-HCl, pH 7.5, 150 mM NaCl)]. After the 8 washes, the bound phage was eluted with 1 ml of 0.2 M glycine-HCl (pH 2.2). In each round, the bound phages were rescued and amplified to make more copies. These brilliant green binding phages were used in a second round of biopanning against pooled disease samples prostate cancer grade group 4 or 5). 96-well plates were incubated overnight with disease samples and then blocked with BSA. Ready plates were used for four rounds of biopanning (three rounds of 10 washes) and the bound phages were harvested and multiplied for luminescence assay testing the functionality in the assay system.

### Example 2

Urine samples were centrifuged 10 000 rpm for 5 min and then diluted 1 to 50 in physiological saline. The faecal samples were processed as follows. Faecal samples in approximately 0.3 ml volume were thawed, added to physiological saline solution until a 1.8 ml volume was reached and subsequently vortexed before sonication 5 minutes in 45°C. Prior to measurement of luminescence, the samples were centrifuged 10 000 rpm for 5 min to remove any excess solid material. The remaining 0.3 ml clear supernatant was dispensed in 15 ml of physiological saline solution. For measurement of urine or faecal samples, each sample was divided in 100 µl volume to a 96 well plate. Finally, 4 µl of 0.1 mM Eu-label, 4 µl of 20 mM brilliant green (CAS Number: 633-03-4) and 4 µl of 1.5×10 10 phage solution was added to the microtiter wells. After 10min of incubation, luminescence emission intensities were measured in a 400 µs window after a 400 µs delay time using a Victor 2 multilabel counter. The absorption measurement was performed with the same device using absorption wavelength 625 nm.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to one embodiment or an embodiment means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment. Where reference is made to a numerical value using a term such as, for example, about or substantially, the exact numerical value is also disclosed.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In the following description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", that is, a singular form, throughout this document does not exclude a plurality.

### REFERENCE SIGNS LIST

- 10: dye-binding phage
- 20: biopanned dye-binding phage
- 30: biopanned biomarker binding bound phages
- 100: Phage Library
- 200: Dye
- 300: Substrate
- 400: individual disease samples
- 500: luminescence assay testing
- 600: stage I biopanning iterations
- 700: stage II biopanning iterations
- 800: Immobilized disease pool samples
- 1000: Biosensing phages

## Claims

1. A method for determining the presence of a biomarker for disease in a pretreated biological sample comprising the steps of
• diluting the sample,
• contacting the sample with a europium label, a terbium label or a samarium label, and a dye,
• contacting the sample with a phage,
• incubating the sample,
• measuring luminescence emission intensity and/or absorption emission intensity, and
• determining the presence of the biomarker based on the measurement of the luminescence emission intensity and/or the absorption emission intensity.

2. A method for visually determining the presence of a biomarker in a biological sample comprising the steps of
• diluting the sample,
• contacting the sample with a europium label, a terbium label or a samarium label, and a dye,
• contacting the sample with a phage,
• incubating the sample,
• observing the colour of the sample, and
• determining the presence of a biomarker based on the colour.

3. The method according to claim 1or 2, wherein the pretreated sample is derived from saliva, sputum, urine, blood or faeces.

4. The method according to any of the preceding claims, wherein the phage is a biosensing phage provided by biopanning in a process comprising,
• contacting a substrate, preferably lignin chips, with a dye to immobilize the dye on the substrate,
• selecting a dye binding phage from a phage library and contacting the substrate chips with the selected phage,
• recovering and amplifying binding phages from the surface of the substrate,
• contacting the recovered binding phages with the label, the dye and pooled disease samples comprising the biomarker for disease,
• harvesting and multiplying the phages bound to the biomarker.

5. The method according to any of the preceding claims, wherein the sample is diluted with physiological saline.

6. The method according to any of the preceding claims, wherein the phage is provided as an aqueous suspension

7. The method according to any of the preceding claims, wherein the sample is incubated for a period in the range of 5 to 15 minutes, preferably 10 minutes at room temperature

8. The method according to any of the preceding claims, wherein the pretreated sample is obtained by centrifuging urine, saliva or sputum, at a speed in the range of 8000 - 12000 rpm, for a period of 5 to 20 minutes and recovering a supernatant.

9. The method according to any of claims 1 to 7, wherein the pretreated sample is obtained by adding 1 part faeces to 5 parts physiological saline, vortexing the mixture, sonicating the mixture for a period of 5 to 20 minutes at a temperature in the range of 40 - 50°C, centrifuging at speed in the range of 8000 - 12000, for a period of 5 to 20 minutes, recovering a supernatant.

10. The method according to any of claims 1 to 7 wherein the pretreated sample is obtained by diluting blood serum or blood plasma in physiological saline in a ratio ranging from 1:10 to 1:1000 by volume, centrifuging at a speed in the range of 8000 to 12000 rpm for a period of 5 to 20 minutes and recovering a supernatant.

11. The method according to any of the preceding claims, wherein the dye is selected from the group consisting of brilliant green, nile blue A and methylene blue.

12. The method according to any of the preceding claims, wherein the label is selected from the group consisting of europium chloride, terbium chloride and samarium chloride.

13. A kit for visually determining the presence of a biomarker for disease in a biological sample said kit comprising:
• means for taking a sample,
• means for preparing the sample,
• means for filtering the sample,
• a receptacle containing a label, a dye, and a phage for determining the presence of a biomarker for disease, and
• a colour scale for comparing a test result to a control.
